Europäisches Patentamt

European Patent Office

Office européen des brevets

(19)

(11) Numéro de publication: **0 202 694**
**B1**

(12) # FASCICULE DE BREVET EUROPEEN

(45) Date de publication du fascicule du brevet:
**13.12.89**

(51) Int. Cl.⁴: **A61B 8/00**, G01S 15/89

(21) Numéro de dépôt: **86200628.5**

(22) Date de dépôt: **15.04.86**

(54) **Appareil d'examen de milieux par échographie ultrasonore.**

(30) Priorité: **19.04.85 FR 8505952**

(43) Date de publication de la demande:
**26.11.86 Bulletin 86/48**

(45) Mention de la délivrance du brevet:
**13.12.89 Bulletin 89/50**

(84) Etats contractants désignés:
**BE DE FR GB SE**

(56) Documents cités:
EP-A- 0 043 158
US-A- 4 063 549

E.A. ASH: "ACOUSTICAL IMAGING",
vol. 12, 1982, pages 413-422, Plenum Press, New York,
US; M. AUPHAN et al.: "Tissue ultrasonic attenuation
well modelized by a mellin-convolution"

(73) Titulaire: **LABORATOIRES D'ELECTRONIQUE ET DE
PHYSIQUE APPLIQUEE L.E.P., 3, Avenue Descartes,
F-94450 Limeil-Brévannes(FR)**

(84) Etats contractants désignés: **FR**

(73) Titulaire: **N.V. Philips' Gloeilampenfabrieken,
Groenewoudseweg 1, NL-5621 BA Eindhoven(NL)**

(84) Etats contractants désignés: **BE DE GB SE**

(72) Inventeur: **Nicolas, Jean-Marie, Société Civile
S.P.I.D. 209 rue de l'Université, F-75007 Paris(FR)**
Inventeur: **Bernatets, Jean-Luc, Société Civile
S.P.I.D. 209 rue de l'Université, F-75007 Paris(FR)**

(74) Mandataire: **Landousy, Christian et al, Société Civile
S.P.I.D. 209, Rue de l'Université, F-75007 Paris(FR)**

**Description**

La présente invention concerne un appareil d'examen de milieux et notamment de tissus biologiques par échographie ultrasonore, comprenant au moins un transducteur ultrasonore associé à un étage d'émission répétée d'ondes ultrasonores vers le milieu à explorer et à un étage de réception des échos renvoyés vers le transducteur par les obstacles rencontrés par ces ondes dans ledit milieu, et, dans ledit étage de réception, un dispositif de transformation de signal échographique.

Le brevet des Etats-Unis d'Amérique US-A 4 016 750 décrit un échographe ultrasonore comprenant, de façon classique, un transducteur ultrasonore associé à un étage d'émission d'ondes ultrasonores et à un étage de réception d'échos comprenant, pour la restitution d'images représentatives de régions du milieux exploré, un circuit d'affichage. Entre le transducteur et ce circuit d'affichage sont prévus un certain nombre de circuits intermédiaires, dont un dispositif composé d'une part d'un amplificateur à gain variable et d'autre part d'un filtre variable. Cet amplificateur et ce filtre assurent respectivement la correction du gain et la correction des caractéristiques de filtrage en fonction de la distance parcourue par les ondes ultrasonores dans le milieu exploré.

Le but de l'invention est également de proposer un échographe ultrasonore corrigeant la diminution de l'intensité du signal ultrasonore et l'atténuation sélective des hautes fréquences avec la distance parcourue, mais dont la structure et le fonctionnement sont fondamentalement différents de ceux qui apparaissent dans le document cité.

A cet effet, l'appareil d'examen selon l'invention est caractérisé en ce que:

(A) ledit dispositif opère par détermination de la transformée de Mellin de ce signal échographique, ladite détermination incluant une conversion linéaire/logarithmique d'échelle temporelle;

(B) l'étage de réception comprend également un dispositif de calcul de fonction de correction par transformation de fonction d'atténuation, opérant par calcul de la valeur inverse de la transformée de Mellin de la fonction d'atténuation associée à une valeur d'atténuation correspondant à au moins une région du milieu à explorer;

(C) l'étage de réception comprend aussi un dispositif de traitement composé en série:
  (1) d'un circuit de multiplication des sorties respectives des dispositifs de transformation prévus en (A) et (B);
  (2) d'un circuit de calcul de la transformée de Mellin inverse du signal ainsi obtenu;

(D) l'étage de réception comprend encore un dispositif de traitement du signal de sortie de ce circuit de calcul de la transformée de Mellin inverse, pour la restitution d'images et/ou d'informations représentatives de ladite région explorée.

La structure ainsi proposée repose sur le principe suivant, à savoir qu'une correction globale des deux effets pernicieux de l'atténuation est effectivement obtenue par la réalisation pratique, dans l'étage de réception de l'appareil, d'une approximation de la transformation mathématique inverse de la transformation intégrale dite convolution de Mellin (cette transformation intégrale est étudiée dans l'article "Tissue ultrasonic attenuation well modelized by a Mellin-convolution" de M. Auphan et J.M. Nicolas paru dans Acoustical Imaging, vol.12, édité par Plenum Publishing Corporation, 1982).

Le brevet des Etats-Unis d'Amérique n° 4 063 549 et la demande de brevet européen EP-A0 043 158 décrivent bien l'un et l'autre un appareil d'examen de milieux par échographie ultrasonore comprenant notamment, dans son étage de réception, un dispositif de transformation de signal échographique, mais cette transformation est une transformation de Fourier et n'est donc pas capable, par nature, de décorréler les effets de l'atténuation dans les milieux explorés, ce qui est au contraire obtenu dans le cas de la présente invention.

Les particularités et avantages de cette invention apparaîtront maintenant dans la description qui suit et dans les dessins annexés, donnés à titre d'exemples non limitatifs et dans lesquels:

– la figure 1 montre un exemple de réalisation de l'appareil selon l'invention;
– la figure 2 montre un mode de réalisation particulier du dispositif de transformation de signal échographique, et les figures 3 à 5 des variantes de réalisation de ce dispositif;
– la figure 6 montre un mode de réalisation particulier du dispositif de transformation de fonction d'atténuation;
– la figure 7 montre dans le dispositif de traitement un mode de réalisation particulier du circuit de calcul de transformée de Mellin inverse, et les figures 8 et 9 deux variantes de ce dispositif de traitement;
– la figure 10 montre une variante de réalisation du dispositif de transformation de fonction d'atténuation et du dispositif de traitement.

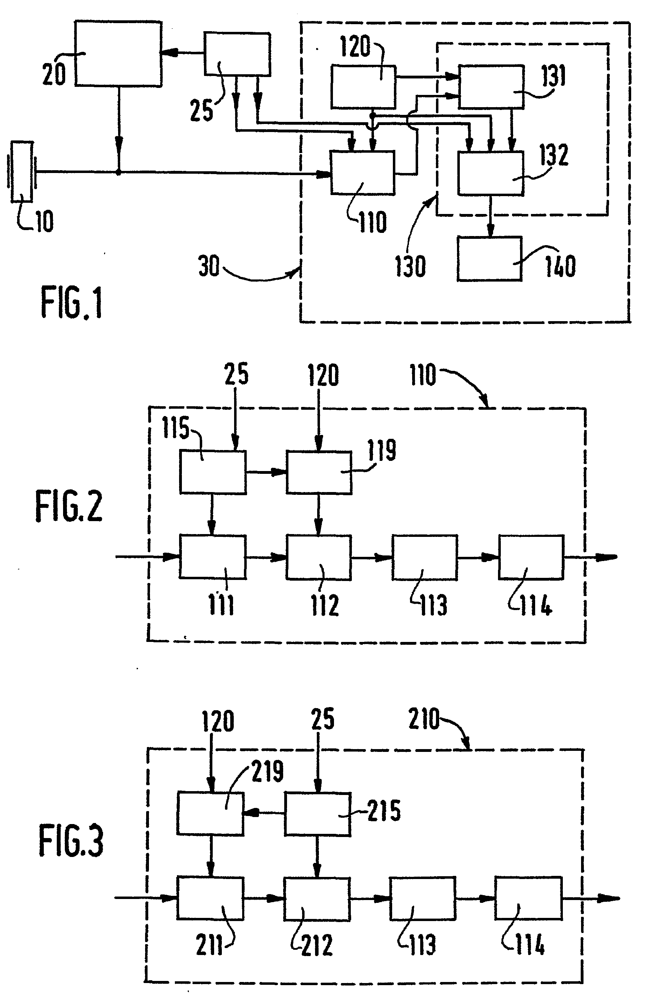

L'appareil représenté sur la figure 1 comprend un transducteur ultrasonore 10 qui, dans l'exemple ici décrit, est destiné à permettre à la fois l'émission d'ondes ultrasonores vers le milieu à examiner par échographie et la réception des échos renvoyés vers lui par les obstacles rencontrés par ces ondes dans la région effectivement explorée. Bien entendu, pour, un déroulement satisfaisant de cet examen, un milieu intermédiaire de transmission est prévu entre le transducteur et la région explorée, mais pour la simplification de la figure ni ladite région ni ce milieu de couplage intermédiaire ne sont représentés. Par ailleurs, on n'utilise dans le cas présent qu'un seul transducteur ultrasonore, mais l'invention est présente de la même manière lorsqu'on utilise des dispositifs tels que sondes, barrettes, réseaux de transducteurs élémentaires pour réaliser les fonctions de conversion d'énergie électrique en énergie ultrasonore et réciproquement.

Les ondes ultrasonores fournies par le transducteur en mode émetteur correspondent à des signaux électriques délivrés par un étage d'émission 20 commandant de façon classique – et donc non décrite en détail ici – une émission répétée, en général périodique, des ondes vers le milieu à explorer; la fréquence de cette émission est couramment comprise entre 1 et 5 kilohertz, et celle des ondes ultrasonores entre 1 et 10 mégahertz, ces indications ne constituant cependant pas des limites impératives. Les échos ultrasonores renvoyés par les différents obstacles qu'ont pu rencontrer les ondes ultrasonores dans la région explorée sont alors reçus par le transducteur 10 en mode récepteur qui, lui-même, fournit les signaux électriques correspondant à ces échos à un étage de réception 30.

Un circuit d'interface non représenté peut être inséré entre le transducteur 10, l'étage d'émission 20 et l'étage de réception 30, notamment pour éviter l'aveuglement de ce dernier par l'étage d'émission. On a par contre représenté, et désigné par la référence 25, le circuit d'horloge qui contrôle le fonctionnement séquentiel des étages, de tels circuits d'horloge étant cependant déjà connus et réalisés dans de nombreux appareils d'échographie, et notamment dans le document cité précédemment.

Conformément à l'invention, l'étage de réception 30 de l'appareil comprend un certain nombre de circuits spécifiques qui sont les suivants : d'une part un dispositif 110 de transformation de signal échographique, destiné à calculer la transformée de Mellin de ce signal, d'autre part un dispositif 120 de calcul de fonction de correction par transformation de fonction d'atténuation, destiné à calculer la valeur inverse de la transformée de Mellin de la fonction d'atténuation associée à une valeur d'atténuation déterminée (précisée ci-dessous), puis un dispositif de traitement 130, destiné à délivrer un signal échographique corrigé en ce qui concerne l'atténuation, et un dispositif de restitution 140, pour le traitement du signal de sortie du dispositif 130.

Le dispositif de traitement 130 comprend lui-même un circuit 131 de multiplication des sorties respectives des dispositifs de transformation 110 et 120 et un circuit 132 de calcul de la transformée de Mellin inverse du signal résultant de cette multiplication.

Dans le cas présent, le dispositif de restitution 140 est un dispositif de visualisation, mais ce pourrait être également, selon l'utilisation envisagée, tout autre dispositif capable de convertir les signaux de sortie du circuit 132 en informations qualitatives ou quantitatives. En effet, selon la volonté des opérateurs, on peut souhaiter soit la restitution d'images des régions explorées, soit la fourniture d'informations qualitatives ou quantitatives représentatives de ces régions explorées, soit une combinaison de ces diverses fonctions. Dans tous les cas, le choix du dispositif de restitution ne constitue pas une limitation de l'invention.

Dans l'exemple décrit jusqu'à présent, la région explorée est une tranche de milieu dans laquelle la valeur de l'atténuation est sensiblement constante et supposée connue sous la forme d'une valeur moyenne, et le dispositif de calcul 120 calcule la transformée de Mellin de cette valeur moyenne à laquelle est associée la fonction d'atténuation.

Dans un mode préférentiel de réalisation, représenté sur la figure 2, le dispositif 110 de transformation de signal échographique comprend, en série, successivement un convertisseur analogique-numérique 111, un circuit 112 de multiplication du signal ainsi numérisé par une rampe de tension linéaire, un circuit 113 d'interpolation par conversion linéaire-logarithmique d'échelle temporelle, et un circuit 114 de calcul de la transformée de Fourier du signal résultant de l'interpolation. L'origine temporelle du signal en rampe est fixée par un circuit 119 de détermination de référence temporelle, qui tient compte du décalage entre le transducteur et la zone dont on veut l'image ou la représentation, et de détermination, si nécessaire, de la ou des valeurs d'atténuation du milieu. Connaissant la valeur moyenne de l'atténuation entre le transducteur et la zone dont on veut l'image ou la représentation, cette référence temporelle est en fait le temps de vol entre ce transducteur et cette zone, multiplié par cette valeur moyenne et divisé par la valeur moyenne de l'atténuation dans la région explorée proprement dite. Une connexion reliant le dispositif 120 au dispositif 110 assure effectivement la transmission de la valeur moyenne de l'atténuation entre le transducteur et la zone explorée. Deux connexions complémentaires entre le dispositif 120 et le circuit 132 d'une part, entre le circuit d'horloge 25 d'autre part et ce même circuit 132 assurent la fourniture audit circuit 132 de cette valeur moyenne d'atténuation et de la référence temporelle $t_0$. Le circuit d'horloge 115 du convertisseur 111 est à échelle linéaire (les impulsions qu'il délivre sont donc régulièrement espacées) et c'est à partir de ce circuit d'horloge qu'est ici généré le signal en rampe proportionnel au temps. Le circuit d'horloge 25 assure comme on l'a vu plus haut la commande de ce circuit 115, ou des autres circuits d'horloge de l'étage de réception, mentionnés dans les variantes de réalisation décrites ci-dessous.

Dans une variante de réalisation représentée sur la figure 3, le dispositif de transformation de signal échographique, référencé 210 cette fois, peut, tout en fonctionnant selon le même principe que le dispositif 110, comprendre les circuits suivants, à savoir un circuit analogique 211 de multiplication du signal échographique par un signal en rampe proportionnel au temps, un convertisseur analogique-numérique 212, dont le circuit d'horloge 215 est également à échelle linéaire et fournit comme précédemment le signal en rampe au circuit de multiplication, puis les circuits 113 et 114 comme précédemment. Un circuit de détermination de référence temporelle 219 est associé au circuit d'horloge 215.

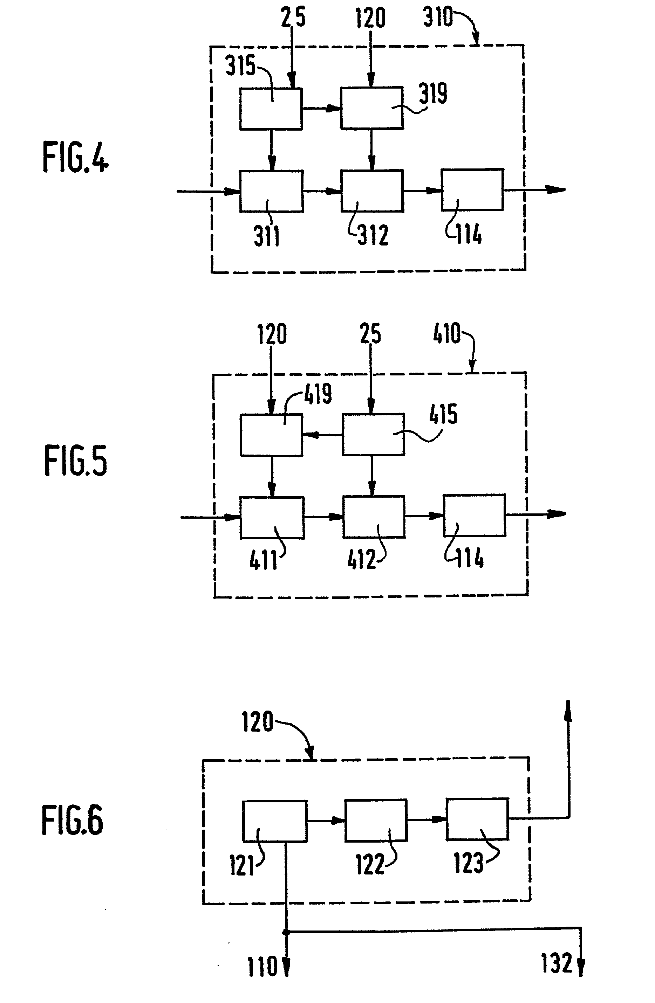

Deux autres variantes de réalisation du dispositif de transformation de signal échographique, référencé cette fois 310 ou 410 respectivement, sont représentées sur les figures 4 et 5. Le dispositif 310, représenté sur la figure 4, comprend en série un convertisseur analogique-numérique 311 du signal échographique, un circuit 312 de multiplication du signal numérisé par un signal proportionnel à Log(t−$t_0$), $t_0$ étant la référence temporelle déjà mentionnée, et le circuit 114. Le dispositif 410, représenté sur la figure 5 comprend un circuit 411 de multiplication du signal échographique, un convertisseur analogique-numérique 412 du signal ainsi obtenu, et le circuit 114 à nouveau. Dans l'un et l'autre cas, le circuit d'horloge, 315 ou 415, du convertisseur est à échelle logarithmique. Comme précédemment, un circuit 319 ou 419 de détermination de référence temporelle est associé au circuit d'horloge 315 ou 415 respectivement.

Le dispositif 120 de calcul de fonction de correction par transformation de fonction d'atténuation associé à ces dispositifs 110, ou 210, ou 310, ou 410 peut également être réalisé de différentes manières, et notamment selon le mode de réalisation de la figure 6. Dans ce cas, il comprend en série d'abord un circuit 121 de prédétermination d'une valeur d'atténuation correspondant à une région du milieu à explorer (par exemple par simple affichage de la valeur moyenne supposée connue plus haut dans la description), et de prédétermination d'une valeur d'atténuation correspondant à la zone entre le transducteur et le milieu exploré (par exemple par affichage également). C'est cette dernière valeur moyenne d'atténuation qui est fournie à la fois au circuit 110 et au circuit 132 (ou aux circuits qui en constituent des variantes). Le dispositif 120 comprend aussi un circuit 122 de calcul de la fonction d'atténuation associée à cette valeur, et un circuit 123 de calcul de la valeur inverse de la transformée de Mellin de cette fonction.

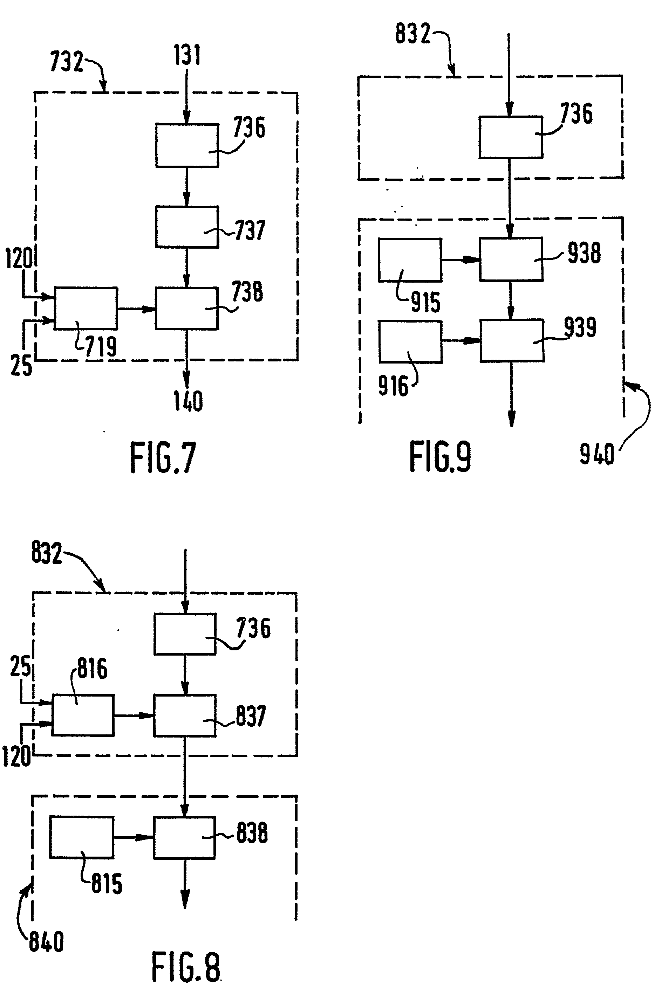

Dans le dispositif de traitement 130 associé par exemple à l'un des dispositifs 110, 210, 310, 410 de transformation de signal échographique décrits précédemment ainsi qu'au dispositif 120 de transformation de fonction d'atténuation également décrit ci-dessus, le circuit de calcul de transformée de Mellin inverse peut être réalisé de la façon suivante. Etant référencé 732, il comprend alors (voir la figure 7) un circuit 736 de calcul de la transformée de Fourier inverse du signal de sortie du circuit de multiplication 131, un circuit 737 d'interpolation par conversion logarithmiquelinéaire d'échelle temporelle, et un circuit 738 de division du signal ainsi obtenu par un signal en rampe proportionnel au temps et d'origine temporelle déterminée comme précédemment à l'aide d'un circuit 719 de détermination d'origine temporelle identique au circuit 119. Dans une première variante de réalisation représentée sur la figure 8, ces circuits 737 et 738 peuvent être remplacés par un circuit 837 de division du signal délivré par le circuit 736 par un signal proportionnel à Log $(t-t_0)$ et généré par le circuit 816. Le dispositif de restitution référencé 840 comprend alors un convertisseur numérique analogique 838 du signal de sortie de ce circuit de division 837, le circuit d'horloge 815 de ce convertisseur étant à échelle exponentielle. Dans une deuxième variante de réalisation représentée sur la figure 9, on prévoit en sortie du circuit 736 un convertisseur numérique-analogique 938 dont le circuit d'horloge 915 est là encore à échelle exponentielle, suivi d'un circuit 939 de division du signal analogique ainsi obtenu par un signal proportionnel à exp$(t-t_0)$ et généré par le circuit 916.

Bien entendu, la présente invention n'est pas limitée aux exemples de réalisation qui viennent d'être décrits et représentés dans quelques variantes.

D'autres variantes peuvent encore être proposées sans pour cela sortir du cadre de l'invention.

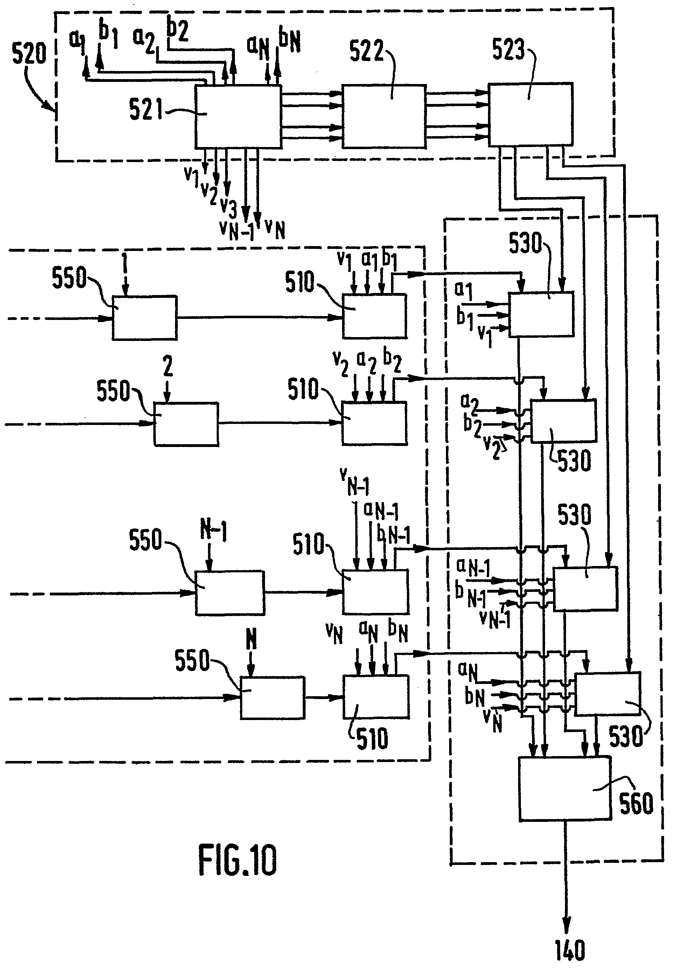

En particulier, la figure 10 montre une variante de réalisation 520 du dispositif de transformation de fonction d'atténuation, ainsi que le dispositif de traitement. Ce dispositif de transformation de fonction d'atténuation 520 comprend un circuit 521 de prédétermination de toute une série de valeurs $a_1$, $a_2$, ..., $a_N$ d'atténuation correspondant respectivement, là encore au moins en moyenne, à des régions du milieu à explorer successives le long de l'axe de propagation des ondes ultrasonores, et de prédétermination d'une série de valeurs $b_1$, $b_2$, ..., $b_N$ de valeurs d'atténuation moyenne correspondant respectivement aux zones comprises entre le transducteur et les régions du milieu à explorer. Le circuit 521 peut être notamment un des appareils d'examen de milieux par échographie ultrasonore décrits par la demanderesse dans deux précédentes demandes de brevet français FR-A 2 514 910 et FR-A 2 534 707.

Le circuit 521 délivre alors d'une part un nombre entier N de valeurs d'atténuation des régions à explorer, d'autre part N valeurs d'atténuation moyenne correspondant donc à la valeur moyenne de l'atténuation entre le transducteur et ces régions à explorer, et enfin N valeurs temporelles $v_1$, $v_2$, ..., $v_N$ d'indication des instants correspondant aux limites desdites régions le long de l'axe de propagation concerné. Un circuit 522 calcule les N fonctions d'atténuation respectivement associées à ces N valeurs d'atténuation, et un circuit 523 calcule les valeurs inverses des transformées de Mellin desdites fonctions.

Dans le cas de cette réalisation de la figure 10, l'appareil comprend N portes 550 commandées par les N valeurs temporelles de sortie du circuit de prédétermination 521, chaque porte étant commandée respectivement par l'une de ces valeurs. Les N portes 550 sont suivies en série de N dispositifs 510 de transformation de signaux échographique, et ces N voies (550, 510) sont en parallèle sur la sortie du transducteur 10 en mode réception (ou sur la sortie du circuit d'interface s'il en est prévu un entre le transducteur, l'étage d'émission et l'étage de réception). L'appareil comprend par ailleurs N dispositifs individuels de traitement 530, suivis d'un additionneur 560. Chaque dispositif 510 reçoit une valeur temporelle $v_1$ à $v_N$ et deux valeurs d'atténuation $a_1$ à $a_N$ et $b_1$ à $b_N$, de même que chaque dispositif 530. La sortie de l'additionneur 560 est reliée à l'entrée du dispositif de restitution 140 déjà prévu dans les modes de réalisation précédents.

## Revendications

1. Appareil d'examen de milieux et notamment de tissus biologiques par échographie ultrasonore, comprenant au moins un transducteur ultrasonore (10) associé à un étage (20) d'émission répétée d'ondes ultrasonores vers le milieu à explorer et à un étage (30) de réception des échos renvoyés vers le transducteur par les obstacles rencontrés par ces ondes dans ledit milieu, et, dans ledit étage de réception; un dispositif de transformation de signal échographique, caractérisé en ce que:

(A) ledit dispositif opère par détermination de la transformée de Mellin de ce signal échographique, ladite détermination incluant une conversion linéaire/logarithmique d'échelle temporelle;

(B) l'étage de réception comprend également un dispositif de calcul de fonction de correction par transformation de fonction d'atténuation, opérant par calcul de la valeur inverse de la transformée de Mellin de la fonction d'atténuation associée à une valeur d'atténuation correspondant à au moins une région du milieu à explorer;

(C) l'étage de réception comprend aussi un dispositif de traitement (130) composé en série;

(1) d'un circuit (131) de multiplication des sorties respectives des dispositifs de transformation prévus en (A) et (B);

(2) d'un circuit (132), de calcul de la transformée de Mellin inverse du signal ainsi obtenu;

(D) l'étage de réception comprend encore un dispositif (140) de traitement du signal de sortie de ce circuit de calcul de la transformée de Mellin inverse, pour la restitution d'images et/ou d'informations représentatives de ladite région explorée.

2. Appareil selon la revendication 1, caractérisé en ce que le dispositif de transformation de signal échographique (110)comprend en série successivement:

(1) un convertisseur analogique-numérique du signal échographique, dont le circuit d'horloge est à échelle linéaire,

(2) un circuit de multiplication du signal numérisé par un signal en rampe proportionnel au temps;

(3) un circuit d'interpolation opérant ladite conversion linéaire-logarithmique d'échelle temporelle;

(4) un circuit de calcul de la transformée de Fourier du signal ainsi obtenu.

3. Appareil selon la revendication 1, caractérisé en ce que le dispositif de transformation de signal échographique (210) comprend en série successivement:

(1) un circuit de multiplication du signal échographique par un signal en rampe proportionnel au temps;

(2) un convertisseur analogique-numérique du signal ainsi obtenu, dont le circuit d'horloge est à échelle linéaire;

(3) un circuit d'interpolation opérant ladite conversion linéaire-logarithmique d'échelle temporelle;

(4) un circuit de calcul de la transformée de Fourier du signal ainsi obtenu.

4. Appareil selon la revendication 1, caractérisé en ce que le dispositif de transformation de signal échographique (310) comprend en série successivement:

(1) un convertisseur analogique-numérique du signal échographique, dont le circuit opère ladite conversion linéaire-logarithmique;

(2) un circuit de multiplication du signal numérisé par un signal proportionnel à $Log(t-t_0)$, où $t_0$ est

une référence temporelle tenant compte de la zone qui sépare le transducteur de la zone dont on veut l'image ou la représentation et de la ou des valeurs d'atténuation du milieu dans cette zone de séparation;

(3) un circuit de calcul de la transformée de Fourier du signal ainsi obtenu.

5. Appareil selon la revendication 1, caractérisé en ce que le dispositif de transformation de signal échographique (410) comprend en série successivement:

(1) un circuit de multiplication du signal échographique par un signal en rampe proportionnel au temps;

(2) un convertisseur analogique-numérique du signal ainsi obtenu, dont le circuit d'horloge opère ladite conversion linéaire-logarithmique.

(3) au moins un circuit de calcul de la transformée de Fourier du signal ainsi converti.

6. Appareil selon l'une des revendications 4 et 5, caractérisé en ce que le dispositif (120), de transformation de fonction d'atténuation comprend en série successivement:

(1) un circuit de prédétermination d'une valeur d'atténuation correspondant au moins en moyenne à une région du milieu à explorer;

(2) un circuit de calcul de la fonction d'atténuation associée à cette valeur;

(3) un circuit de calcul de la transformée de Mellin de cette fonction d'atténuation.

7. Appareil selon la revendication 6, caractérisé en ce que le circuit (132) de calcul de transformée de Mellin inverse du dispositif de traitement comprend:

(1) un circuit de calcul de la transformée de Fourier inverse du signal de sortie du circuit de multiplication;

(2) un circuit d'interpolation par conversion logarithmique-linéaire d'échelle temporelle;

(3) un circuit de division du signal ainsi obtenu par un signal en rampe proportionnel au temps.

8. Appareil selon la revendication 6, caractérisé en ce que le circuit (132) de calcul de transformée de Mellin inverse du dispositif de traitement comprend:

(1) un circuit de calcul de transformée de Fourier inverse;

(2) un circuit de division du signal ainsi obtenu par un signal proportionnel à $Log(t-t_0)$;

et en ce que le dispositif de restitution (140) comprend un convertisseur numérique-analogique du signal de sortie du circuit de division, le circuit d'horloge de ce convertisseur étant à échelle exponentielle.

9. Appareil selon la revendication 6, caractérisé en ce que le circuit (132) de calcul de transformée de Mellin inverse du dispositif de traitement comprend un circuit de calcul de la transformée de Fourier inverse;

et en ce que le dispositif de restitution (140) comprend:

(1) un convertisseur numérique-analogique du signal de sortie de ce circuit de calcul, le circuit d'horloge de ce convertisseur étant à échelle exponentielle;

(2) un circuit de division du signal analogique ainsi obtenu par un signal proportionnel à exp($t-t_0$).

10. Appareil selon l'une des revendications 7 à 9, caractérisé en ce que le dispositif (520) de transformation de fonction d'atténuation comprend:

(1) un circuit de prédétermination d'une série de valeurs d'atténuation correspondant respectivement, au moins en moyenne, à des régions du milieu à explorer successives le long de l'axe de propagation des ondes ultrasonores, ledit circuit délivrant d'une part un nombre entier N de valeurs d'atténuation des régions à explorer, d'autre part N valeurs d'atténuation moyenne correspondant à la valeur moyenne de l'atténuation entre le transducteur et ces régions à explorer, enfin N valeurs temporelles d'indication des instants correspondant aux limites desdites régions à explorer le long de l'axe de propagation;

(2) un circuit de calcul des N fonctions d'atténuation respectivement associées auxdites valeurs d'atténuation;

(3) un circuit de calcul des transformées de Mellin desdites fonctions d'atténuation;

en ce que le dispositif de transformation de signal échographique comprend N voies en parallèle comprenant elles-mêmes en série:

(1) N portes (550) commandées par lesdites valeurs temporelles de sortie du circuit de prédétermination;

(2) N dispositifs individuels (510) de transformation de signal échographique;

et en ce qu'il est également prévu:

(1) N dispositifs de traitement (530);

(2) un additionneur (560) des sorties de ces N dispositifs de traitement, la sortie de cet additionneur étant reliée à l'entrée du dispositif de restitution.

## Claims

1. An apparatus for examining media, notably biological tissues, by means of ultrasonic echography, which apparatus comprises at least one ultrasonic transducer (10) which is connected to a stage (20) for the repeated transmission of ultrasonic waves to the medium to be scanned, to a stage (30) for receiving the echoes which are reflected in the direction of the transducer by the obstacles encountered by these waves in said medium, and also comprises, included in said receiver stage, a device for transforming the echographic signal, characterized in that:

(A) said device operates by determination of the Mellin transform of this echographic signal, which determination includes a linear/logarithmic conversion of the time scale;

(B) the receiver stage also comprises a device for calculating a correction function by transformation of the attenuation function, operation taking place by calculating the inverse value of the Mellin transform of the attenuation function associated with an attenuation value corresponding to at least one region of the medium to be scanned;

(C) the receiver stage also comprises a processing device (130) which is composed of a series connection of:

(1) a circuit (131) for multiplying the respective output signals of the transformation devices sub (A) and (B);

(2) a circuit (132) for calculating the inverse Mellin transform of the signal thus obtained;

(D) the receiver stage also comprises a device (140) for processing the output signal of this circuit for calculating the inverse Mellin transform for the display of images and/or data representative of said region scanned.

2. An apparatus as claimed in Claim 1, characterized in that the device (110) for transforming the echographic signal includes a series connection of successively:

(1) an analog-to-digital converter for the echographic signal, the clock circuit thereof having a linear scale;

(2) a circuit for multiplying the digitized signal by a ramp signal which is proportional to the time;

(3) an interpolation circuit which performs said linear/logarithmic conversion of the time scale;

(4) a circuit for calculating the Fourier transform of the signal thus obtained.

3. An apparatus as claimed in Claim 1, characterized in that the device (210) for transforming the echographic signal includes a series connection of successively:

(1) a circuit for multiplying the echographic signal by a ramp signal which is proportional to the time;

(2) an analog-to-digital converter for the signal thus obtained, the clock circuit thereof having a linear scale;

(3) an interpolation circuit for performing said linearlogarithmic conversion of the time scale;

(4) a circuit for calculating the Fourier transform of the signal thus obtained.

4. An apparatus as claimed in Claim 1, characterized in that the device (310) for transforming the echographic signal includes a series connection of successively:

(1) ana analog-to-digital converter for the echographic signal, the clock circuit thereof performing said linearlogarithmic conversion;

(2) a circuit for multiplying the digitized signal by a signal which is proportional to $\log(t-t_0)$, $t_0$ being a time reference taking into account the zone situated between the transducer and the zone for which an image or a representation is desired, and also taking into account the attenuation value or values of the medium in this separating zone;

(3) a circuit for calculating the Fourier transform of the signal thus obtained.

5. An apparatus as claimed in Claim 1, characterized in that the device (410) for transforming the echographic signal includes a series connection of successively:

(1) a circuit for multiplying the echographic signal by a ramp signal which is proportional to the time;

(2) an analog-to-digital converter for the signal thus obtained, the clock circuit thereof performing said linear-logarithmic conversion;

(3) at least one circuit for calculating the Fourier transform of the signal thus converted.

6. An apparatus as claimed in Claim 4 or 5, characterized in that the device (120) for transforming the attenuation function includes a series connection of successively:

(1) a circuit for predetermining an attenuation value which corresponds at least on average to a region of the medium to be scanned;

(2) a circuit for calculating the attenuation function associated with this value;

(3) a circuit for calculating the Mellin transform of this attenuation function.

7. An apparatus as claimed in Claim 6, characterized in that the circuit (132) for calculating the inverse Mellin transform of the processing device includes:

(1) a circuit for calculating the inverse Fourier transform of the output signal of the multiplier circuit;

(2) a circuit for interpolation by linear-logarithmic conversion of the time scale;

(3) a circuit for dividing the signal thus obtained by a ramp signal which is proportional to the time.

8. An apparatus as claimed in Claim 6, chararterized in that the circuit (132) for calculating the inverse Mellin transform of the processing device includes:

(1) a circuit for calculating the inverse Fourier transform;

(2) a circuit for dividing the signal thus obtained by a signal which is proportional to $\log(t-t_0)$;

and in that the display device (140) includes a digital-to-analog converter for the output signal of the divider circuit, the clock circuit of said converter having an exponential scale.

9. An apparatus as claimed in Claim 6, characterized in that the circuit (132) for calculating the inverse Mellin transform of the processing circuit includes a circuit for calculating the inverse Fourier transform;

and in that the display device (140) includes:

(1) a digital-to-analog converter for the output signal of said arithmetic circuit, the clock circuit of said converter having an exponential scale;

(2) a circuit for dividing the analog signal thus obtained by a signal which is proportional to $\exp(t-t_0)$.

10. An apparatus as claime din any one of the Claims 7 to 9, characterized in that the device (520) for transforming the attenuation function includes:

(1) a circuit for predetermining a series of attenuation values which correspond, at least on average, to respective regions of the medium to be scanned which are successively situated along the axis of propagation of the ultrasonic waves, said circuit supplying an integer number of N attenuation values of the regions to be scanned, N mean attenuation values, corresponding to the mean value of the attenuation between the transducer and said regions to be scanned, and N time values which indicate the instants which correspond to the boundaries of said regions to be scanned along the axis of propagation.;

(2) a circuit for calculating the N attenuation functions which are associated with the respective attenuation values;

(3) a circuit for calculating the Mellin transforms of said attenuation functions ;

in that the device for transforming the echographic signal includes N parallel channels which themselves include in series:

(1) N gates (550) which are controlled by said time values which originate from the output of the predetermination circuit;

(2) N separate devices (510) for transforming the echographic signal;

and in that there are also provided:

(1) N processing devices (530);

(2) an adder (560) for the output signals of said N processing devices, the output of said adder being connected to the input of the display device.

## Patentansprüche

1. Gerät zur Untersuchung von Medien und insbesondere von biologischen Geweben mittels Ultraschall-Echographie, mit wenigstens einem Ultraschall Wandler (10), der einer Stufe (20) zum wiederholten Aussenden von Ultraschallwellen in das zu untersuchende Medium und einer Stufe (30) zum Empfangen von Echos zugeordnet ist, die durch Abprallen dieser Wellen an in diesem Medium befindlichen Gegenständen an den Meßwertumsetzer gelangen, und mit einer Umwandlungsvorrichtung des echographischen Signals in dieser Empfangsstufe, dadurch gekennzeichnet, daß

(A) die Umwandlungsvorrichtung die Mellin-Transformierte dieses echographischen Signals bestimmt, und diese Bestimmung eine linear/logarithmische Umwandlung des Zeitmaßstabs einschließt,

(B) die Empfangsstufe weiter einen Korrekturfunktionsberechner durch Schwächungswertumwandlung enthält, die den invertierten Wert der Mellin-Transformierte der Schwächungsfunktion berechnet, die einem Schwächungswert entsprechend wenigstens einem Gebiet des zu untersuchenden Mediums zugeordnet ist,

(C) die Empfangsstufe noch eine Verarbeitungseinrichtung (130) mit der Reihenschaltung aus

(1) einer Multiplikationsschaltung (131) der jeweiligen Ausgangssignale der unter (A) und (B) vorgesehenen Wandler,

(2) einem Berechnungsschaltung (132) der invertierten Mellin Transformierte des so gewonnenen Signals enthält,

(D) die Empfangsstufe außerdem eine Bearbeitungseinrichtung (140) des Ausgangssignals dieser Berechnungsschaltung der invertierten Mellin-Transformierte zum Wiederherstellen von Bildern und/oder von das untersuchte Gebiet darstellenden Informationen enthält.

2. Gerät nach Anspruch 1, dadurch gekennzeichnet, daß die Umwandlungsvorrichtung (110) des echographischen Signals aufeinanderfolgend in Reihenschaltung folgende Elemente enthält:

(1) einen Analog/Digital-Wandler des echographischen Signals, dessen Taktschaltung einen linearen Maßstab hat,

(2) eine Schaltung zum Multiplizieren des digitalisierten Signals mit einem Signal mit zeitproportionaler Steigung,

(3) eine Interpolationsschaltung zum Durchführen der linear/logarithmischen Umwandlung des Zeitmaßstabs,

(4) eine Berechnungsschaltung der Fourier-Transformierte des so gewonnenen Signals.

3. Gerät nach Anspruch 1, dadurch gekennzeichnet, daß die Umwandlungsvorrichtung (210), des echographischen Signals aufeinanderfolgend in Reihenschaltung folgende Elemente enthält:

(1) eine Schaltung zum Multiplizieren des echographischen Signals mit einem Signal mit zeitproportionaler Steigung,

(2) einen Analog/Digital-Wandler des so gewonnenen Signals, dessen Taktschaltung einen linearen Maßstab hat,

(3) eine Interpolationsschaltung zum Durchführen der linear/logarithmischen Umwandlung des Zeitmaßstabs,

(4) eine Berechnungsschaltung der Fourier-Transformierte des so gewonnenen Signals.

4. Gerät nach Anspruch 1, dadurch gekennzeichnet, daß die Umwandlungsvorrichtung (210) des echographischen Signals aufeinanderfolgend in Reihenschaltung folgende Elemente enthält:

(1) einen Analog/Digital-Wandler des echographischen Signals, dessen Schaltung die linear/-logarithmische Umsetzung versorgt,

(2) eine Schaltung zum Multiplizieren des digitalisierten Signals mit einem Signal proportional $\text{Log}(t-t_0)$, worin $t_0$ eine Zeitreferenz unter Berücksichtigung der Zone, die den Wandler von der Zone trennt, deren Bild oder Darstellung gewünscht wird, und des oder der Schwächungswerte des Mediums in dieser Trennzone ist,

(3) eine Berechnungsschaltung der Fourier-Transformierte des so gewonnenen Signals.

5. Gerät nach Anspruch 1, dadurch gekennzeichnet, daß die Umwandlungsvorrichtung (410) des echographischen Signals aufeinanderfolgend in Reihenschaltung folgende Elemente enthält:

(1) eine Schaltung zum Multiplizieren des echographischen Signals mit einem Signal mit zeitproportionaler Steigung,

(2) einen Analog/Digital-Wandler des so gewonnenen Signals, dessen Taktschaltung die linear/logarithmische Umsetzung versorgt,

(3) wenigstens eine Berechnungsschaltung der Fourier-Transformierte des so gewonnenen Signals.

6. Gerät nach Anspruch 4 oder 5, dadurch gekennzeichnet, daß der Schwächungsfunktionswandler (120) aufeinanderfolgend in Reihenschaltung folgende Elemente enthält:

(1) eine Vorbestimmungsschaltung eines Schwächungswerts entsprechend wenigstens im Mittel einem Gebiet des zu untersuchenden Mediums,

(2) eine Berechnungsschaltung der diesem Wert zugeordneten Schwächung,

(3) eine Berechnungsschaltung der Mellin-Transformierte dieser Schwächung.

7. Gerät nach Anspruch 6, dadurch gekennzeichnet, daß die Berechnungsschaltung (132) der invertierten Mellin-Transformierte der Bearbeitungseinrichtung folgende Elemente enthält:

(1) eine Berechnungsschaltung der invertierten Fourier-Transformierte des Ausgangssignals der Multiplikationsschaltung,

(2) eine Interpolationsschaltung durch logarithmisch/lineare Umwandlung des Zeitmaßstabs,

(3) eine Schaltung zum Teilen des so gewonnenen Signals durch ein Signal mit zeitproportionaler Steigung.

8. Gerät nach Anspruch 6, dadurch gekennzeichnet, daß die Berechnungsschaltung (132) der invertierte Mellin-Transformierte der Bearbeitungseinrichtung folgende Elemente enthält:

(1) eine Berechnungsschaltung der invertierten Fourier-Transformierte,

(2) eine Schaltung zum Teilen des so gewonnenen Signals durch ein Signal proportional $\text{Log}(t-t_0)$, und daß die Wiederherstellungseinrichtung (140) einen Digital/Analog-Wandler des Ausgangssignals der Teilerschaltung enthält, wobei die Taktschaltung dieses Wandlers einen Exponentialmaßstab hat.

9. Gerät nach Anspruch 6, dadurch gekennzeichnet, daß die Berechnungsschaltung (132) der invertierten Mellin-Transformierte der Bearbeitungseinrichtung eine Berechnungsschaltung der invertierten Fourier-Transformierte enthält, und daß die Wiederherstellungseinrichtung (140) folgendes enthält:

(1) einen Digital/Analog-Wandler des Ausgangssignals dieser Berechnungsschaltung, wobei die Taktschaltung dieses Wandlers einen Exponentialmaßstab hat,

(2) eine Schaltung zum Teilen des so gewonnenen analogen Signals durch ein Signal proportional $\exp(t-t_0)$.

10. Gerät nach einem der Ansprüche 7 bis 9, dadurch gekennzeichnet, daß der Schwächungsfunktionswandler (520) folgendes enthält:

(1) eine Vorbestimmungsschaltung einer Schwächungswertfolge entsprechend wenigstens im Mittel jeweils Gebieten des aufeinanderfolgenden zu untersuchenden Mediums entlang der Fortpflanzungsachse der Ultraschallwellen, wobei die Schaltung einerseits eine ganze Zahl von N Schwächungswerten der zu untersuchenden Gebiete, andererseits N mittlere Schwächungswerte entsprechend dem mittleren Schwächungswert zwischen dem Wandler und diesen zu untersuchenden Gebieten und schließlich N Zeitwerte zum Anzeigen der Zeitpunkte entsprechend der Begrenzungen dieser zu untersuchenden Gebiete entlang der Fortpflanzungsachse liefert,

(2) eine Berechnungsschaltung der N Schwächungsfunktionen, die jeweils den Schwächungswerten zugeordnet sind,

(3) eine Berechnungsschaltung der Mellin-Transformierten dieser Schwächungsfunktionen, daß die Umwandlungsvorrichtung des echographischen Signals N parallele Kanäle enthält, die selbst wieder in Reihenschaltung folgendes enthalten:

(1) N Tore (550), die durch die zeitlichen Ausgangswerte der Vorbestimmungsschaltung gesteuert werden,

(2) N Einzeleinrichtungen (510) zum Umwandeln echographischer Signale, und daß ebenfalls vorgesehen sind:

(1) N Bearbeitungseinrichtungen (530),

(2) ein Addierer (560) der Ausgangssignale dieser N Bearbeitungseinrichtungen, wobei der Ausgang dieses Addierers mit dem Eingang der Wiederherstellungseinrichtung verbunden ist.

FIG.1

FIG.2

FIG.3

FIG.4

FIG.5

FIG.6

FIG.7

FIG.9

FIG.8

FIG.10